# FASCICULE DE BREVET EUROPEEN

(11) **EP 0 750 920 B1**
(45) Date de publication et mention de la délivrance du brevet: **03.12.2003**
(21) Numéro de dépôt: 96401440.1
(22) Date de dépôt: 28.06.1996
(51) Int. Cl.: A61N 1/365

(54) **Dispositif médical implantable actif, notamment stimulateur cardiaque, asservi à au moins un paramètre physiologique**
Aktive implantierbare medizinische Vorrichtung, insbesondere Herzschrittmacher, der durch mindestens einen physiologischen Parameter gesteuert wird
Active implantable medical device, in particular pacemaker, controlled by at least one physiological parameter

(30) Priorité: 30.06.1995 FR 9507928
(43) Date de publication de la demande: 02.01.1997
(73) Titulaire: ELA MEDICAL (Société anonyme), 92541 Montrouge (FR)
(72) Inventeur: Bonnet, Jean-Luc, 92170 Vanves (FR); Geroux, Laurence, 92350 Le Plessis-Robinson (FR)
(74) Mandataire: Dupuis-Latour, Dominique

(56) Documents cités:
- EP-A- 0 249 820
- EP-A- 0 331 309
- EP-A- 0 452 732
- WO-A-92/03182
- US-A- 5 014 702

## Description

La présente invention concerne les "dispositifs médicaux implantables actifs" tels que définis par la directive 90/385/CEE du 20 juin 1990 du Conseil des communautés européennes, notamment les stimulateurs cardiaques ou défibrillateurs, dont le fonctionnement est asservi à un paramètre recueilli à l'aide d'un capteur.

À cet égard, bien que dans la suite de la description on fasse principalement référence au cas d'un stimulateur cardiaque, l'invention est applicable de façon générale à une très grande variété de dispositifs électroniques.

De tels appareils sont connus pour adapter leurs actions, par exemple la fréquence de stimulation dans le cas d'un stimulateur cardiaque, à la valeur mesurée ou calculée d'un paramètre représentatif des besoins métaboliques du porteur de l'appareil.

Le EP-A-0 089 014 décrit l'utilisation de la mesure de la fréquence respiratoire pour faire varier la fréquence instantanée de la stimulation cardiaque. De multiples paramètres, tels que la ventilation-minute, la mesure de la saturation d'oxygène dans le sang, la température ou l'accélération ont été utilisés comme paramètres d'asservissement.

Dans les cas des stimulateurs cardiaques, tous ces systèmes concourent à augmenter la fréquence des impulsions de stimulation lorsque l'on détecte une activité croissante du patient porteur de l'appareil, et à diminuer cette fréquence jusqu'à une valeur plancher en cas de diminution de cette activité, tout particulièrement durant les phases de repos du patient.

Certains capteurs, tels que celui décrit dans le EP-A-0 493 220, donnent une représentation adéquate des besoins métaboliques du patient, mais présentent des temps de réponse longs, si bien qu'ils ne sont pas adaptés pour faire varier la fréquence de stimulation lors d'efforts de courte durée.

D'autres capteurs, tels que celui décrit dans le FR-A-2 685 642, présentent un temps de réponse court et permettent de détecter rapidement un changement de l'activité du porteur de l'appareil ; ils sont particulièrement adaptés à la détection des débuts de phases d'effort.

À cet égard, dans un souci de simplification, on nommera "capteur d'effort" le capteur mesurant le paramètre le plus physiologique et "capteur d'activité" le capteur à temps de réponse plus court.

On a déjà proposé d'associer les informations issues de deux capteurs de types différents afin de profiter des avantages de chacun d'eux. Une telle association est décrite notamment dans les US-A-5 014 702 et US-A-5 065 759.

Le US-A-5 014 702 décrit une méthode où le capteur d'effort sert à confirmer les informations délivrées par le capteur d'activité. C'est le capteur d'activité qui détermine de façon prépondérante la fréquence de stimulation. Le capteur d'effort confirme ou infirme la présence d'un exercice physique et, en cas de confirmation, fait varier la fréquence de stimulation d'une valeur prédéfinie (15 cpm)

Le US-A-5 065 759 décrit une méthode où, inversement, la prédominance du réglage de la fréquence est donnée au capteur d'effort, les indications de ce dernier capteur étant pondérées suivant le sens et l'amplitude des variations enregistrées par l'autre capteur. Toutefois, ces variations et ces amplitudes sont déterminées par comparaison à des courbes de référence spécifiques au porteur et préenregistrées dans l'appareil.

Le EP-A- 0 452 732 décrit un dispositif dans lequel les indications des deux capteurs sont utilisées concurremment, avec application à chaque capteur d'un facteur de pondération variable. Le signal servant à l'asservissement est ainsi une combinaison linéaire, variable de manière adaptative, des signaux délivrés conjointement par les deux capteurs.

Le EP-A- 0 249 820, quant à lui, décrit un dispositif comprenant des moyens pour sélectionner l'un ou l'autre des deux capteurs et asservir le fonctionnement du dispositif sur le capteur ainsi sélectionné à un instant donné. Cette sélection est opérée pendant des durées prédéterminées, de manière figée, après détection d'un début d'exercice par un capteur d'accélération.

L'invention à pour objet de pallier l'ensemble des inconvénients associés à ces techniques antérieurement proposées, grâce à un dispositif médical implantable actif, notamment un stimulateur cardiaque perfectionné, asservi à un paramètre recueilli à l'aide d'un capteur, qui offre :
- une association améliorée des deux capteurs d'effort et d'activité, grâce notamment à une réciprocité des confirmations respectives des signaux captés, c'est-à-dire par "surveillance croisée" de l'indication de chaque capteur par l'autre capteur ;
- un pilotage adaptatif de la fréquence cardiaque, opéré par celui des capteurs qui aura été choisi à un moment donné par le système, et non par un capteur prédéterminé, en profitant du fait que capteur d'effort et capteur d'activité ont des réponses différentes pour utiliser au mieux les caractéristiques propres de chacun des capteurs en fonction du stade d'évolution de l'activité du patient : début d'activité, activité confirmée, récupération après un effort, etc. ;
- un fonctionnement et une gestion du système entièrement indépendants de tout coefficient pondérateur à programmer ou de toute courbe de référence introduite ou déterminée pour chaque patient ;
- un suivi des indications du premier capteur à avoir détecté un début d'exercice ;
- un filtrage des artefacts éventuels, avec détection et prise en compte des "trous" d'information éventuels d'un capteur (ces trous d'information, qui seront définis plus précisément par la suite, peuvent être considérés comme des baisses erratiques du signal d'information délivré par l'un ou l'autre des capteurs et qui ne résultent pas d'une baisse d'activité).

Le dispositif de l'invention est du type genéral divulgué par le EP-A-0 249 820 précité. Pour atteindre les buts précités, il comporte les caractéristiques énoncées dans la partie caractérisante de la revendication 1.

Les sous-revendications visent des modes de mise en oeuvre avantageux de l'invention.

D'autres caractéristiques et avantages de l'invention apparaîtront à la lecture de la description détaillée ci-dessous, faite en référence aux dessins annexés.
La figure 1 est un organigramme général du fonctionnement du dispositif.
La figure 2 détaille le sous-ensemble de la figure 1 relatif à l'analyse de l'état des capteurs.
Les figures 3 et 4 sont des tableaux indiquant les actions prises par le dispositif en fonction de l'état des capteurs respectifs.
Les figures 5 à 7 illustrent le comportement de la fréquence de stimulation conformément aux enseignements de l'invention.

Dans la description détaillée qui va suivre, on prendra l'exemple d'un stimulateur cardiaque asservi à un paramètre physiologique. Cette application n'est cependant pas limitative, et les enseignements de l'invention sont directement applicables à d'autres types de dispositifs médicaux implantables actifs.

De même, bien que l'on fera référence à deux capteurs seulement, on peut prévoir dans une version plus élaborée un nombre supérieur de capteurs, multiplexés ou combinés entre eux, permettant d'asservir le fonctionnement du dispositif à une pluralité de paramètres physiologiques différents.

Enfin, les types de capteurs décrits ici (à savoir un capteur de ventilation-minute pour le capteur d'effort et un capteur d'accélération pour le capteur d'activité) n'est également pas limitatif, et d'autres types de capteurs peuvent être envisagés.

De surcroît, les caractéristiques de fonctionnement, notamment les temps de réponse, de ces deux types de capteurs respectifs sont différentes, ce qui permet avantageusement d'en cumuler les avantages lorsque l'on combine leurs fonctionnements : le capteur de ventilation-minute présente un temps de réponse assez long mais délivre un signal réellement représentatif des besoins métaboliques du patient ; en revanche, l'accéléromètre a un temps de réponse extrêmement faible, mais il ne donne pas d'indication sur les véritables besoins métaboliques du patient.

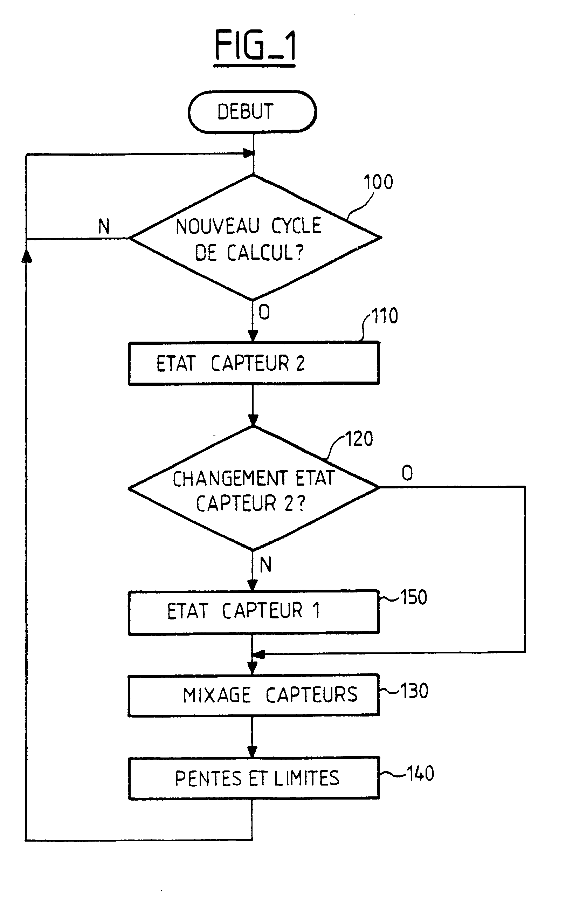

L'algorithme illustré figure 1 permet de calculer une fréquence d'asservissement (fréquence de stimulation cardiaque) à partir de deux capteurs dont les informations sont mesurées régulièrement par le stimulateur, typiquement toutes les 1,5625 seconde pour l'accélération et tous les cycles respiratoires pour la ventilation-minute. La mesure de ces informations est en elle-même classique et cet aspect ne sera pas décrit ici.

Ces informations sont transformées en une fréquence que l'on appellera par la suite "fréquence capteur", ou plus spécifiquement "fréquence MV" pour le capteur de ventilation-minute et "fréquence G" pour le capteur d'accélération.

De même, dans la suite, on appellera "Capteur 1" le capteur d'activité, c'est-à-dire le capteur permettant de détecter rapidement un début ou une fin d'activité mais ne donnant pas de niveau proportionnel à l'effort (typiquement un capteur d'accélération, également désigné "G"), et "Capteur 2" le capteur d'effort, dont le temps de réaction en début et en fin d'activité est plus long que le Capteur 1 mais qui donne un niveau réellement significatif des besoins métaboliques du patient (typiquement un capteur de ventilation-minute, également désigné "MV"). On notera cependant que la différence de temps de réponse entre les capteurs n'est pas forcément très importante, la ventilation-minute étant par exemple connue comme étant un paramètre physiologique présentant en début d'effort une réponse rapide.

L'algorithme général illustré figure 1 correspond à un cycle de base qui est mis en oeuvre systématiquement, par exemple tous les quatre cycles cardiaques (test de boucle 100). On examine alors (étape 110) l'état du Capteur 2 et l'on teste s'il y a eu ou non modification de l'état de ce capteur (étape 120).

Dans l'affirmative, on détermine, en fonction de critères prédéterminés que l'on explicitera plus bas, le capteur qui pilotera l'asservissement (étape 130), avec application (140) de pentes et de limites pour éviter des variations trop rapides de la fréquence cardiaque (selon des algorithmes, en eux-mêmes connus, de gestion de la croissance ou de la décroissance du signal cardiaque pour un stimulateur asservi).

Dans la négative, on analyse l'état du Capteur 1 pour déterminer s'il y a eu transition d'états (étape 150) et l'on poursuit l'algorithme avec les étapes 130 et 140 précitées.

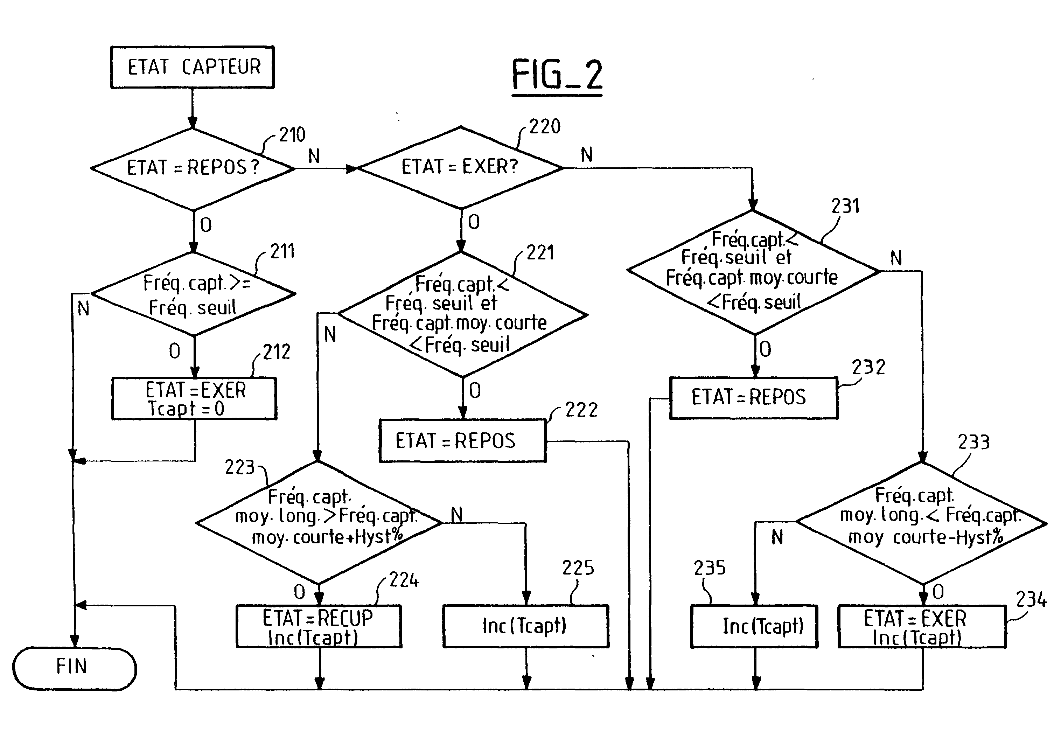

L'organigramme de la figure 2 concerne la détermination de l'état du Capteur 2 (étape 110 de l'organigramme de la figure 1) ; on verra plus bas que cet organigramme s'applique également, moyennant quelques différences mineures, au cas du Capteur 1 (étape 150 sur la figure 1).

Il permet de définir, pour chacun des capteurs, trois états différents :
- "REPOS", lorsque l'activité détectée est inférieure à un seuil prédéfini (phase dite "de repos") ;
- "EXER", lorsque l'activité, supérieure à un seuil prédéfini, est croissante ou stable (phase dite "d'exercice") ; et
- "RÉCUP", lorsque l'activité, supérieure au seuil prédéfini, est strictement décroissante (phase dite "de récupération").

Par ailleurs, on définit différentes fréquences capteur (les fréquences étant définies de la même façon pour chacun des deux capteurs) :
- "Fréquence Capteur Instantanée" : fréquence capteur déterminée par la relation d'asservissement (relation qui lie la mesure de l'information délivrée par le capteur à la fréquence de stimulation) ;
- "Fréquence Capteur" : moyenne des N dernières valeurs de Fréquence Capteur Instantanée (N étant différent pour chaque capteur) ;
- "Fréquence Capteur Moyenne Courte" : moyenne des X dernières valeurs de Fréquence Capteur Instantanée (X étant différent pour chaque capteur ) ;
- "Fréquence Capteur Moyenne Longue" : moyenne des Y dernières valeurs Fréquence Capteur Instantanée (Y étant différent pour chaque capteur, et Y > X).
- "Fseuil" : fréquence égale à la fréquence de base du stimulateur, plus 6 % pour le Capteur 2, et 12 % pour le Capteur 1 (valeurs bien entendu non limitatives).

On applique alors les règles suivantes, telles que mises en oeuvre par l'algorithme de la figure 2 :
- si le capteur est à REPOS (étape 210) :
   * si Fréquence Capteur ≥ Fseuil (étape 211), alors le capteur passe à EXER (étape 212) ;
   * sinon il reste à REPOS.
- si le capteur est à EXER (étape 220) :
   * si Fréquence Capteur < Fseuil et si Fréquence Capteur Moyenne Courte < Fseuil, alors le capteur passe à REPOS (étape 222) ;
   * si Fréquence Capteur Moyenne Longue ≥ Fréquence Capteur Moyenne Courte + Hyst (étape 223), "Hyst" étant un paramètre d'hystérésis, alors le capteur passe à RÉCUP (étape 224) ;
   * sinon le capteur reste à EXER.
- si le capteur est à RÉCUP :
   * si Fréquence Capteur < Fseuil et si Fréquence Capteur Moyenne Courte < Fseuil (étape 231), alors le capteur passe à REPOS (étape 232) ;
   * si Fréquence Capteur Moyenne Longue < Fréquence Capteur Moyenne Courte - Hyst (étape 233), alors le capteur passe à EXER (étape 234) ;
   * sinon le capteur reste à RÉCUP.

Un autre paramètre que l'on détermine simultanément ici est un temps d'activité de capteur ("Tcapt"), mis à jour à chaque fois que l'on met à jour l'état de ce capteur.

Dans ce mode de réalisation Tcapt est un compteur de passages, mais il peut être également un compteur de temps. Tcapt est égal à zéro si le capteur est à REPOS. Il est incrémenté (étapes 224, 225, 235 et 234), par exemple, du nombre de cycles cardiaques depuis la dernière mise à jour dans les états EXER et RÉCUP. Tcapt permettra de limiter le temps d'asservissement sur un capteur qui détecte une activité qui n'est pas confirmée par l'autre capteur.

Pour la détermination de l'état du Capteur 1 (étape 150 de la figure 1), l'organigramme est le même que celui de la figure 2, à la seule différence des étapes 221 et 231, où le test est effectué uniquement par rapport à Fréquence Seuil.

Si l'on revient à l'organigramme de la figure 1, pour simplifier le fonctionnement, on ne permet qu'à un seul capteur de changer d'état par cycle de calcul, avec donc la nécessité de définir une priorité entre les capteurs.

Dans l'exemple illustré, le Capteur 2 est le capteur prioritaire, c'est-à-dire que l'on teste d'abord l'état de celui-ci, et ce n'est que s'il n'a pas changé d'état que l'on teste l'état du Capteur 1 ; en revanche, si l'état a changé, on ne teste pas l'état du Capteur 1.

Cette restriction n'est pas indispensable, mais elle a cependant pour avantage de diminuer le nombre de transitions possibles d'un cycle au suivant, et donc de simplifier la gestion de l'étape 130.

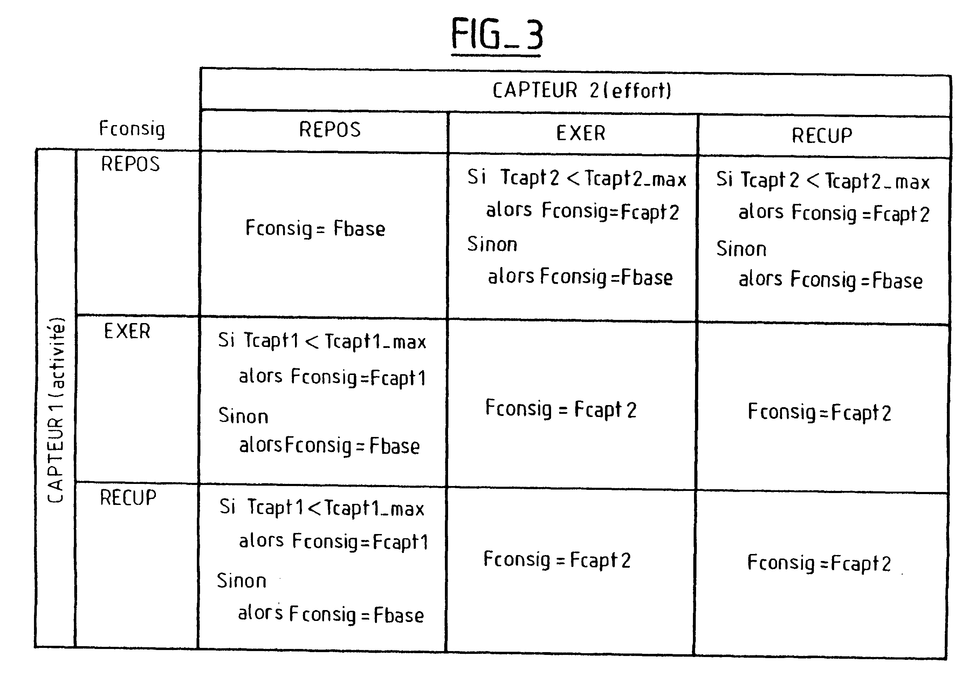

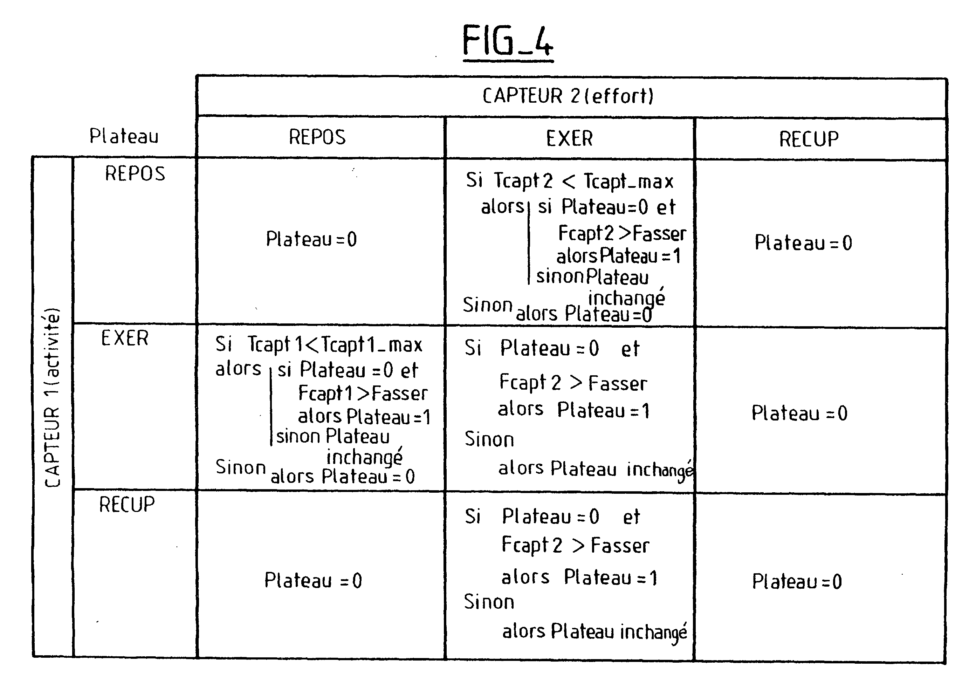

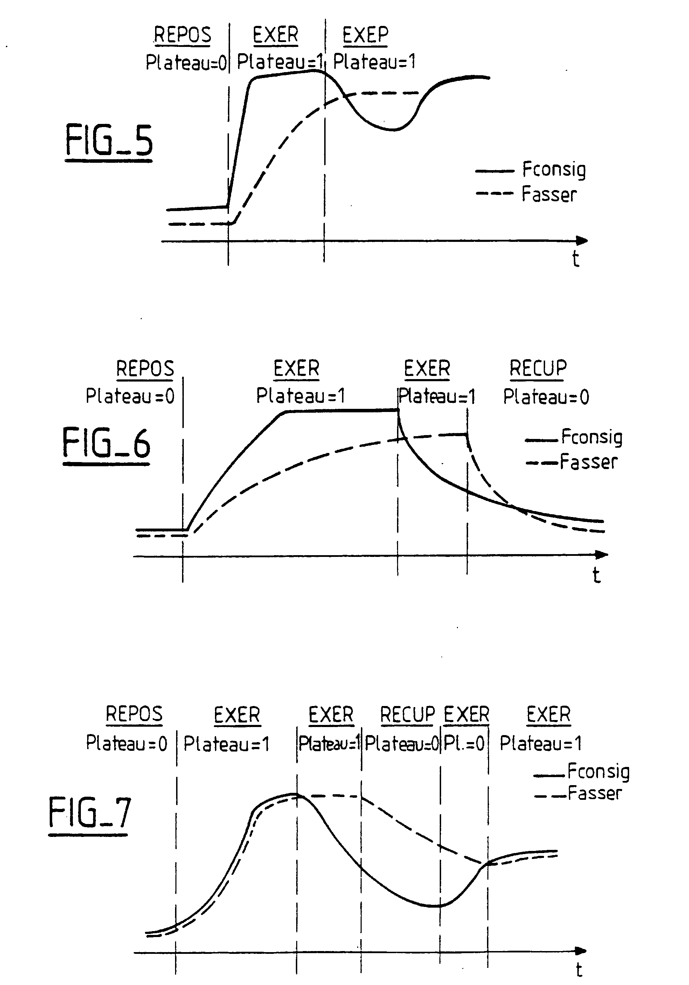

Le mixage des capteurs effectué à l'étape 130 a pour but de déterminer deux variables : la fréquence de consigne Fconsig, qui est la fréquence cible que doit atteindre le stimulateur (tableau de la figure 3) et la variable dénommée "Plateau", qui permet de gérer les trous d'information (tableau de la figure 4). La combinaison de ces deux variables, ainsi que l'application des pentes et des limites, donnera la fréquence d'asservissement (Fasser) à appliquer (étape 140), qui est illustrée sur les exemples des figures 5 à 7).

Dans l'exemple du système illustré à deux capteurs et trois états par capteur, on est en présence de neuf combinaisons possibles, qui constituent les éléments d'une matrice {3,3} pour chacun des tableaux des figures 3 et 4.

Pour chaque combinaison, on détermine une fréquence de consigne (Fconsig), c'est-à-dire une fréquence vers laquelle devra se diriger la fréquence d'asservissement. Fconsig peut prendre trois valeurs :
- "Fbase" : la fréquence de base programmée ;
- "Fcapt1" : la fréquence d'asservissement du Capteur 1 ;
- "Fcapt2" : la fréquence d'asservissement du Capteur 2.

On se référera au tableau de la figure 3 pour le détail du mixage des capteurs, dont le principe général consiste à utiliser dès que possible l'information du Capteur 2.

On remarquera la symétrie de la première ligne et de la première colonne. Fconsig prend la valeur de la fréquence du capteur qui a détecté une activité ou une récupération durant la phase de vérification de durée Tcapt (avec Tcapt < Tcapt_max, valeur prédéfinie maximale pour chacun des capteurs), sinon Fconsig prend la valeur Fbase.

Partant du principe que le Capteur 2 est plus représentatif de la demande métabolique du patient, Fconsig prend la valeur indiquée par ce capteur dès que ce dernier indique un état en activité (EXER ou RÉCUP) confirmé par le Capteur 1 : colonnes 2 et 3 et lignes 2 et 3 de la matrice des possibilités.

Parallèlement à la détermination de Fconsig, le système détermine la valeur d'un variable dénommée "Plateau".

L'objet de cette variable est de gérer la variation de la fréquence d'asservissement en présence d'un "trou" d'information de l'un des capteurs.

On a vu plus haut dans la définition des états qu'EXER et RÉCUP correspondent à une activité, EXER étant une activité croissante et RÉCUP étant une activité décroissante. La notion de croissance et de décroissance est implicite dans les conditions données ci-dessus : en effet, le principe permettant de dire si une activité est croissante ou décroissante est basée sur la comparaison de deux moyennes de fréquences capteur, l'une à court terme (Fréquence Capteur Moyenne Courte) l'autre à long terme (Fréquence Capteur Moyenne Longue).

Ce principe des "croisements de moyenne" (qui a déjà été décrit dans le FR-A-2 671 012 au nom de la demanderesse et ne sera pas exposé plus en détail) a pour effet de discriminer entre des trous d'information et une véritable fin d'activité du patient (passage en REPOS) ou un passage à une activité plus faible (passage en RÉCUP). On peut donc définir un trou d'information comme une diminution de courte durée de Fréquence Capteur alors que l'état du capteur est toujours EXER. Dans ce cas, la fréquence d'asservissement ne doit pas être modifiée et l'on doit donc appliquer un "plateau" de fréquence.

Plus précisément, la variable "Plateau" prend la valeur '0' lorsque la fréquence d'asservissement peut descendre (pas de trou : il s'agit d'une fin d'effort) et la valeur '1' lorsque la fréquence d'asservissement ne doit pas descendre (plateau durant un trou).

Le tableau de la figure 4 expose la manière dont on détermine la valeur de Plateau en fonction de la case de la matrice où se situe le système. On se référera à la figure 4 pour la détermination cas par cas de la valeur de Plateau selon les états des différents capteurs et la valeur de cette même variable à l'évaluation précédente.

La fréquence effective d'asservissement Fasser est calculée à partir de la nouvelle valeur Fconsig et de la valeur de Plateau (étape 140). Le principe est le suivant :
- Si la fréquence de consigne Fconsig (donnée par l'un des capteurs, Fcapt suivant le tableau de la figure 3) est supérieure à la fréquence d'asservissement actuelle Fasser, le système a détecté une activité d'un ou des deux capteurs. On augmente la fréquence d'asservissement.
- Si la fréquence de consigne est égale à la fréquence de base, le système a détecté un repos, ou a invalidé l'activité détectée par un seul capteur. La fréquence d'asservissement est diminuée.
- Si la fréquence de consigne est supérieure à la fréquence de base et inférieure à la fréquence d'asservissement actuelle, le système se trouve dans un cas où il doit différencier entre une baisse d'activité et un trou d'information du capteur utilisé. Comme Plateau est mis à 1 lorsqu'un plateau de fréquence doit être observé (voir ci-dessus), si Plateau = 1 le système n'a pas encore diagnostiqué de baisse d'activité (l'état du capteur utilisé est EXER) et la fréquence d'asservissement n'est pas modifiée. En revanche, si Plateau = 0, le système a diagnostiqué une baisse d'activité et la fréquence d'asservissement est diminuée.

On peut résumer les modalités de cette fonction d'asservissement par les règles suivantes :
- si Fasser < Fconsig, alors Fasser est augmentée (avec application des pentes et limites mentionnées ci-devant) ;
- si Fasser > Fconsig, alors on teste la valeur de Fconsig :
   - si Fconsig = Fbase (fin d'activité confirmée), alors Fasser descend vers Fbase ;
   - si Fconsig ≠ Fbase, alors on teste Plateau :
      - si Plateau = 1 (trou respiratoire), alors Fasser n'est pas modifiée (plateau) ;
      - si Plateau = 0 (fin ou baisse d'activité), alors Fasser descend vers Fconsig.

On a schématisé sur les figures 5 à 7 divers résultats expérimentaux obtenus par la mise en oeuvre de l'invention.

Ces figures illustrent les variations de Fasser sous diverses conditions de variation de l'information Fconsig.

Sur la figure 5, avant la détection d'une activité par l'un des capteurs, on a Plateau = 0. Ensuite, Fasser tend à rejoindre la valeur indiquée par Fconsig ; Plateau est alors à 1 (passage à la case située 2e ligne, 2e colonne de la matrice de la figure 4). Au moment de la diminution temporaire ("trou d'information") de Fconsig, Plateau = 1 et la fréquence d'asservissement conserve la valeur atteinte au moment du croisement des fréquences Fconsig et Fasser.

La figure 6 illustre le comportement de Fasser durant une phase de RÉCUP précédée par une période d'EXER : la fréquence d'asservissement présente un plateau avant de décroître vers la fréquence indiquée par le capteur.

La figure 7 illustre la cas d'une reprise d'activité après une phase de RÉCUP : dans ce cas, la variable Plateau prend la valeur 0 (passage à la case située 1re ligne, 2e colonne de la matrice de la figure 4) afin de permettre à la fréquence d'asservissement de rejoindre la fréquence indiquée par le capteur.

## Revendications

1. Un dispositif médical implantable actif, notamment un stimulateur cardiaque, susceptible d'un fonctionnement asservi par pilotage d'au moins une fonction du dispositif, notamment d'une fréquence de stimulation cardiaque, par au moins un paramètre physiologique, ce dispositif comportant :
- au moins un capteur d'effort mesurant un paramètre à prépondérance physiologique et délivrant un signal fonction de l'effort développé par un patient porteur du dispositif,
- au moins un capteur d'activité, en particulier un capteur à faible temps de réponse, mesurant un paramètre à prépondérance physique et délivrant un autre signal fonction de l'activité dudit patient, et
- des moyens sélecteurs de l'un ou l'autre des capteurs, fonctionnant en réponse aux signaux de chacun de ces capteurs,
dispositif **caractérisé en ce que**, chacun des capteurs étant apte à délivrer un signal susceptible de prendre une pluralité d'états différents, lesdits moyens sélecteurs de l'un ou l'autre des capteurs sont des moyens aptes à :
. analyser périodiquement la séquence relative des changements d'états successifs des capteurs en fonction de critères prédéterminés intrinsèques au dispositif, et
. sélectionner l'un ou l'autre des capteurs en fonction du résultat de cette analyse,
de manière à asservir, à un moment donné, ladite fonction du dispositif à celui des capteurs sélectionné par les moyens sélecteurs.

2. Le dispositif médical implantable actif de la revendication 1, dans lequel chacun des capteurs est susceptible d'au moins trois états distincts (REPOS, EXER, RÉCUP) fonction de l'activité détectée par le paramètre mesuré par ce capteur, ces trois états correspondant respectivement à une phase de repos, une phase d'exercice et une phase de récupération.

3. Le dispositif médical implantable actif de la revendication 2, dans lequel le capteur passe à l'état (REPOS) correspondant à la phase de repos lorsque le paramètre mesuré est inférieur à un seuil prédéfini.

4. Le dispositif médical implantable actif de la revendication 2, dans lequel le capteur passe à l'état (EXER) correspondant à la phase d'exercice lorsque le paramètre mesuré est supérieur au seuil prédéfini et croissant ou stable.

5. Le dispositif médical implantable actif de la revendication 2, dans lequel le capteur passe à l'état (RÉCUP) correspondant à la phase de récupération lorsque le paramètre mesuré est supérieur au seuil prédéfini et strictement décroissant.

6. Le dispositif médical implantable actif de la revendication 1, dans lequel les moyens sélecteurs comparent entre eux les états pris par les deux capteurs respectifs à un même cycle de mesure donné et associent à chaque combinaison possible d'états une valeur de consigne (Fconsig) pilotant le fonctionnement asservi du dispositif.

7. Le dispositif médical implantable actif de la revendication 6, comportant en outre des moyens de contrôle croisé des indications des capteurs, pour comparer l'état (EXER ou RÉCUP) déterminé par le signal du capteur d'activité et ce même état d'activité tel que déterminé par le signal du capteur d'effort, et pour mettre fin au fonctionnement en mode asservi en cours en cas de non-concordance.

8. Le dispositif médical implantable actif de la revendication 7, dans lequel les moyens de contrôle croisé mettent fin au fonctionnement asservi en cours lorsque ladite non-concordance est constatée pendant un temps supérieur à une durée prédéterminée, ou après un nombre d'itérations de l'étape de comparaison supérieur à une valeur prédéterminée.

9. Le dispositif médical implantable actif de la revendication 1, comportant en outre des moyens de protection contre les ruptures d'information, ces moyens étant des moyens aptes à opérer une discrimination entre une baisse ou fin d'activité et un trou d'information, ne résultant pas d'une baisse d'activité, du signal délivré par l'un ou l'autre des capteurs.

10. Le dispositif médical implantable actif de la revendication 9, dans lequel les moyens de protection contre les ruptures d'information maintiennent constante, en cas de baisse momentanée d'activité, la valeur (Fasser) de ladite fonction pilotant le fonctionnement asservi du dispositif.

11. Le dispositif médical implantable actif de la revendication 10, dans lequel les moyens de protection contre les ruptures d'information mettent en oeuvre une variable d'état binaire déterminée : d'une part par le résultat d'une comparaison entre la valeur (Fasser) de ladite fonction pilotant le fonctionnement asservi du dispositif et une valeur de consigne (Fconsig) de cette même fonction ; d'autre part par la valeur antérieure de la variable d'état ; et en dernier lieu par les états des deux capteurs.

## Patentansprüche

1. Aktive implantierbare medizinische Vorrichtung, insbesondere ein Herzschrittmacher, geeignet zum geregelten Betrieb durch Steuerung mindestens einer Funktion der Vorrichtung, insbesondere einer Frequenz der Herzstimulation, durch mindestens einen physiologischen Parameter, wobei diese Vorrichtung aufweist:
- wenigstens einen Sensor der Anstrengung, der einen überwiegend physiologischen Parameter misst und ein Signal abgibt, das abhängt von der von einem Patienten, der Träger dieser Vorrichtung ist, entwickelten Anstrengung,
- wenigstens einen Sensor der Aktivität, insbesondere einen Sensor mit geringer Antwortzeit, der einen überwiegend physischen Parameter misst und ein anderes Signal abhängig von der Aktivität des besagten Patienten liefert, und
- Einrichtungen zum Auswählen des einen oder des anderen der Sensoren, funktionierend in Antwort auf die Signale jeder dieser Sensoren,
wobei die Vorrichtung **dadurch gekennzeichnet ist, dass** jeder dieser Sensoren dazu geeignet ist, ein Signal zu liefern, das eine Vielzahl verschiedener Zustände annehmen kann, wobei die besagten Einrichtungen zur Auswahl des einen oder des anderen der Sensoren Einrichtungen sind, die geeignet sind:
die relative Sequenz der sukzessiven Zustandsänderungen der Sensoren periodisch zu analysieren abhängig von innerhalb der Vorrichtung vorbestimmten Kriterien der Einrichtungen, und
. abhängig vom Ergebnis dieser Analyse den einen oder den anderen dieser Sensoren auszuwählen,
in der Weise, dass zu einem gegebenen Zeitpunkt die Funktion der Vorrichtung geregelt wird durch denjenigen der Sensoren, der durch die Einrichtungen zur Auswahl ausgewählt ist.

2. Aktive implantierbare medizinische Vorrichtung nach Anspruch 1, bei der jeder der Sensoren empfänglich für wenigstens drei unterschiedliche Zustände (REPOS, EXER, RECUP) ist, abhängig von der Aktivität, die detektiert wird durch den von diesem Sensor gemessenen Parameter, wobei diese drei Zustände jeweils mit einer Phase der Ruhe, einer Phase der Belastung und einer Phase der Erholung entsprechen.

3. Aktive implantierbare medizinische Vorrichtung nach Anspruch 2, bei der der Sensor in den Zustand (REPOS), entsprechend der Phase der Erholung, wechselt, wenn der gemessene Parameter kleiner ist als ein vorgegebener Schwellenwert.

4. Aktive implantierbare medizinische Vorrichtung nach Anspruch 2, bei der der Sensor in den Zustand (EXER), entsprechend der Phase der Belastung, wechselt, wenn der gemessene Parameter größer als ein vorgegebner Schwellenwert und zunehmend oder stabil ist.

5. Aktive implantierbare medizinische Vorrichtung nach Anspruch 2, bei der der Sensor in den Zustand (RECUP), entsprechend der Phase der Erholung, wechselt, wenn der gemessene Parameter größer als ein vorgegebener Schwellenwert und strikt abnehmend ist.

6. Aktive implantierbare medizinische Vorrichtung nach Anspruch 1, bei der die Einrichtungen zur Auswahl zwischen den Zuständen vergleichen, welche die jeweiligen zwei Sensoren in einem gleichen gegebenen Messzyklus einnehmen, und für jede mögliche Kombination der Zustände einen hinterlegten Wert (Fconsig) assoziieren, der den geregelten Betrieb der Vorrichtung steuert.

7. Aktive implantierbare medizinische Vorrichtung nach Anspruch 6, die außerdem Einrichtungen aufweist zur kreuzweisen Kontrolle der Ausgaben der Sensoren, um den Zustand (EXER oder RECUP), der bestimmt wird von dem Signal des Sensors der Aktivität, zu vergleichen mit dem gleichen Zustand der Aktivität, welcher bestimmt wird durch das Signal des Sensors der Anstrengung, und zur Beendigung des Betriebs im geltenden Regelmodus im Falle einer Nichtübereinstimmung.

8. Aktive implantierbare medizinische Vorrichtung nach Anspruch 7, bei der die Einrichtungen zur kreuzweisen Kontrolle den gegenwärtigen geregelten Betrieb beendet, wenn die Nichtübereinstimmung festgestellt wird während einer Zeit, die länger ist als eine vorgegebene Dauer, oder nach einer Anzahl der Iterationen der Vergleichsschritte, die größer ist als ein vorgegebener Wert.

9. Aktive implantierbare medizinische Vorrichtung nach Anspruch 1, außerdem aufweisend Einrichtungen zum Schutz gegen Unterbrechungen der Information, wobei diese Einrichtungen Einrichtungen sind, die dazu geeignet sind, eine Unterscheidung durchzuführen zwischen einem Rückgang oder Ende der Aktivität und einer Lücke der Information des Signals, welches von dem einen oder dem anderen der Sensoren abgegeben wird, und das nicht von einem Rückgang der Aktivität herrührt.

10. Aktive implantierbare medizinische Vorrichtung nach Anspruch 9, bei der die Einrichtungen zum Schutz vor Unterbrechungen der Information im Falle einer vorübergehenden Abnahme der Aktivität den Wert (Fasser) der besagten Funktion, welche die Regelung der Vorrichtung steuert, konstant erhält.

11. Aktive implantierbare medizinische Vorrichtung nach Anspruch 10, bei der die Einrichtungen zum Schutz vor Unterbrechungen der Information eine festgelegte binäre Zustandsvariable verwirklicht: zum einen nach dem Ergebnis eines Vergleichs zwischen dem Wert (Fasser) der besagten Funktion zur Steuerung der Regelung der Vorrichtung und einem hinterlegten Wert (Fconsig) derselben Funktion; zum anderen nach dem früheren Wert der Zustandsvariablen; und in letzter Linie nach den Zuständen der zwei Sensoren.

## Claims

1. An active implantable medical device, in particular a cardiac pacemaker, the operation of which may be enslaved by driving at least one function of the device, in particular a cardiac stimulation frequency, said device including:
- at least one effort sensor measuring a preponderantly physiological parameter and issuing a signal which is a function of the effort developed by a patient bearing the device;
- at least one activity sensor, in particular a fast-response sensor, measuring a preponderantly physical parameter and issuing a further signal which is a function of the effort developed by said patient; and
- means for selecting either one of the sensors, functioning in response to the signals of every of theses sensors,
said device being **characterized in that**, each of the sensors being able to issue a signal which may take a plurality of different states, said means for selecting either one of the sensors are means adapted to:
* periodically analyze the relative sequence of successive state changes of the sensors according to predetermined criteria intrinsic to the device; and
* selecting either one of the sensors as a function of the result of said analysis,
whereby, at a given time, said function of the device is enslaved to that sensor which is selected by the selecting means.

2. The active implantable medical device of claim 1, in which each of the sensors may take at least three distinct states (REPOS, EXER, RECUP) as a function of the activity detected by the parameter measured by this sensor, these three states respectively corresponding to a phase of rest, a phase of exercise and a phase of recovery.

3. The active implantable medical device of claim 2, in which the sensor turns to the state (REPOS) corresponding to the phase of rest when to the measured parameter is less than a predetermined threshold.

4. The active implantable medical device of claim 2, in which the sensor turns to the state (EXER) corresponding to the phase of exercise when the measured parameter is greater than the predetermined threshold and increasing or stable.

5. The active implantable medical device of claim 2, in which the sensor turns to the state (RECUP) corresponding to the phase of recovery when the measured parameter is greater than the predetermined threshold and strictly decreasing.

6. The active implantable medical device of claim 1, in which the selecting means compare the mutual states taken by the respective sensors during a given measurement cycle and associate with each possible combination of states a reference value (Fconsig) which drives the enslaved operation of the device.

7. The active implantable medical device of claim 6, further comprising means to cross-check the indications of the sensors, for comparing the state (EXER or RECUP) determined by the signal of the activity sensor and this same state of activity as determined by the signal of the effort sensor, and for terminating the instant enslaved mode of operation of the device in case of non-matching.

8. The active implantable medical device of claim 7, in which the cross-checking means terminate the instant enslaved mode of operation when said non-matching is found for a duration longer than a predetermined time, or after a number of repetitions of the comparison step which is greater than a predetermined value.

9. The active implantable medical device of claim 1, further comprising means for protection against gaps of information, said means being means adapted to make a discrimination between a decline or end of activity and a gap of information, not being the result of a decline of activity, of the signal issued by either one of the sensors.

10. The active implantable medical device of claim 9, in which the means for protection against gaps of information maintain constant, in a case of a momentary activity decline, the value (Fasser) of said function driving the enslaved operation of the device.

11. The active implantable medical device of claim 10, in which the means for protection against gaps of information make use of a binary state variable determined: on the one hand, by the result of a comparison between the value (Fasser) of said function driving the enslaved operation of the device and a reference value (Fconsig) of said function; on the other hand by the previous value of the state variable; and finally by the states of the two sensors.
